# EUROPÄISCHE PATENTSCHRIFT

(11) **EP 2 656 806 B1**
(45) Veröffentlichungstag und Bekanntmachung des Hinweises auf die Patenterteilung: **20.01.2016**
(21) Anmeldenummer: 13159342.8
(22) Anmeldetag: 15.03.2013
(51) Int. Cl.: A61B 18/00, A61B 18/10, A61B 18/12, A61B 18/14, H01R 13/15

(54) **Buchsenanordnung für ein elektromedizinisches Gerät**
Socket assembly for an electric medical device
Agencement de douille pour un appareil électromédical

(30) Priorität: 26.04.2012 EP 12165760
(43) Veröffentlichungstag der Anmeldung: 30.10.2013
(73) Patentinhaber: ERBE Elektromedizin GmbH, 72072 Tübingen (DE)
(72) Erfinder: Munkelt, Katja, 07629 Hermsdorf (DE)
(74) Vertreter: Rüger, Barthelt & Abel

(56) Entgegenhaltungen:
- US-A- 5 621 256
- US-A- 6 074 386
- US-A1- 2002 128 692
- US-A1- 2005 027 327
- US-B1- 7 035 689

## Beschreibung

Die Erfindung betrifft eine Buchsenanordnung für ein elektromedizinisches Gerät, an das ein elektromedizinisches, vorzugsweise chirurgisches Instrument angeschlossen werden kann. Das Instrument ist mit einem Stecker über die Buchsenanordnung mit dem elektromedizinischen Gerät elektrisch verbindbar. Das Gerät stellt eine Hochfrequenzspannung oder einen Hochfrequenzstrom für das angeschlossene Instrument zur Verfügung. Das Gerät und das Instrument dienen zur Hochfrequenz-Chirurgie. Bei der Hochfrequenz-Chirurgie werden Spannungen und Ströme mit einer Frequenz im Bereich von 200 oder 300 kHz bis zu etwa 4 MHz verwendet.

Problematisch ist dabei, dass die Stecker der elektromedizinischen Instrumente nicht genormt sind und verschiedene Stecker mit mehreren Steckerkontaktstiften existieren, die unterschiedliche Durchmesser und Abstände haben. Dies führt dazu, dass nicht jeder Stecker eines chirurgischen Instruments mit der zugeordneten Buchsenanordnung jedes elektromedizinischen Geräts ohne weiteres verbunden werden kann.

Außerdem existieren chirurgische Instrumente, die für monopolare bzw. bipolare Anwendungen ausgelegt sind. Sie können unterschiedliche Stecker mit einer unterschiedlichen Anzahl von Steckerkontaktstiften aufweisen.

Im Operationssaal ist ein zügiger und reibungsloser Ablauf wichtig. Technische Schwierigkeiten und dadurch bedingte Verzögerungen sind zu vermeiden. Dies setzt auch voraus, dass eine gute mechanische und elektrische Verbindung zwischen dem Stecker eines elektromedizinischen Instruments und der Buchsenanordnung des Geräts hergestellt werden kann.

DE 10 2007 061 483 A1 beschreibt ein Chirurgiegerätesteckersystem mit einer Vielzahl von Steckerbuchsen für Stecker von elektromedizinischen Geräten. Über eine Schaltmatrix können Steckerbuchsen der Buchsenanordnung mit bestimmten Ein- oder Ausgängen des elektromedizinischen Geräts elektrisch verbunden werden. Diese elektrische Verbindung über die Schaltmatrix kann beispielsweise anhand der von einer Datenschnittstelle der Buchsenanordnung empfangenen Daten vorgenommen werden. Die Daten können an die Datenschnittstelle vom Stecker des elektromedizinischen Instruments übermittelt werden. Die Buchsenanordnung des Gerätes führt sozusagen eine automatische Steckererkennung durch und stellt automatisch die notwendigen elektrischen Verbindungen über die Schaltmatrix her. Fehler beim Anschließen des Instruments an das Gerät können auf diese Weise vermieden werden. Allerdings löst die Schaltmatrix noch nicht das Problem, dass unterschiedlich dimensionierte Stecker existieren, die mechanisch und elektrisch sicher verbunden werden müssen.

Eine elektrische Steckverbindung mit einer Buchsenanordnung ist aus DD 214 724 bekannt. Die Buchsenanordnung weist mehrere Hülsen auf, die auf der Anschlussseite zur Aufnahme eines Steckerkontaktstifts über eine die Hülse tragende Platte hinausragen. In dem überstehenden Teil der Hülse ist eine Aussparung vorhanden, in die ein beispielsweise zylindrisches Kontaktelement eingesetzt ist. Das zylindrische Kontaktelement ist über eine gebogene Schenkelfeder schwenkbar gegenüber der Hülse an der Platte gelagert. Auf der der Anschlussseite entgegengesetzten Rückseite ragt die Schenkelfeder aus der Platte heraus und kann dort einen elektrischen Anschluss bilden. Der Steckerkontaktstift berührt nach dem Einschieben in die Hülse das Kontaktelement und ist über das Kontaktelement elektrisch mit der Schenkelfeder verbunden.

Weiter ist aus der US 2002/0128692 A1 eine SteckerBuchsen-Anordnung bekannt, die Rastkugeln enthält. Der Steckerstift weist mindestens eine Umfangsnut auf. In der Wand der Steckbuchse sind an passender Stelle Radialbohrungen angebracht, in denen Rastkugeln sitzen. Diese werden durch ein Element aus einem Memory-Metall nach innen gespannt und greifen bei eingestecktem Steckerstift in dessen Umfangsnut.

Ausgehend von diesem bekannten Stand der Technik kann es als eine Aufgabe der vorliegenden Erfindung angesehen werden, eine Buchsenanordnung für ein elektromedizinisches Gerät zu schaffen, das eine sichere mechanische und elektrische Kontaktierung für unterschiedliche Steckerbauformen und Stecker unterschiedlicher instrumententypen ermöglicht.

Erfindungsgemäß wird eine Buchsenanordnung gemäß Anspruch 1 vorgeschlagen. Sie weist wenigstens eine in einem Gehäuseteil angeordnete Steckerbuchse auf. Als Gehäuseteil dient beispielsweise ein Abschnitt des Gehäuses des elektrcmedizinischen Geräts oder ein separates Buchsengehäuse der Buchsenanordnung, das in das Gerät eingesetzt werden kann. Die wenigstens eine Steckerbuchse weist ein elektrisches erstes Kontaktelement sowie ein elektrisches zweites Kontaktelement auf. Das zweite Kontaktelement ist relativ zum ersten Kontaktelement beweglich gelagert. Die beiden Kontaktelemente sind auf gegenüberliegenden Seiten eines Aufnahmebereichs für einen Steckerkontaktstift angeordnet. Sie befinden sich insbesondere quer zu einer Einsteckrichtung, entlang der der Steckerkontaktstift in die Steckerbuchse einsteckbar ist, mit Abstand zueinander auf diametral entgegengesetzten Seiten. Beide Kontaktelemente begrenzen den Aufnahmebereich für den Steckerkontaktstift in Umfangsrichtung um die Einsteckrichtung vorzugsweise jeweils in einem Umfangsabschnitt. Vorzugsweise sind in Umfangsrichtung gesehen zwischen den beiden Umfangsabschnitten der Kontaktelemente gegenüber dem Inneren des Gehäuses offene Bereiche vorhanden. Durch diese offenen Bereiche ist die Aufnahme von Steckerkontaktstiften mit unterschiedlichen Durchmessern ermöglicht.

Bei einem Ausführungsbeispiel ist der Aufnahmebereich in zumindest zwei axial aneinander anschließende Abschnitte unterteilt. Eine Gehäuseöffnung bildet einen ersten Abschnitt des Aufnahmebereichs. Daran schließt sich ein zweiter Abschnitt des Aufnahmebereichs an, in dem die Kontaktelemente angeordnet sind. Abgesehen von den Kontaktelementen ist der Aufnahmebereich in diesem zweiten Abschnitt vorzugsweise zum Inneren des Gehäuses offen und insbesondere nicht durch hülsenförmige Teile der Steckerbuchse im Durchmesser beschränkt. Vorzugsweise kann sich an den zweiten Abschnitt ein dritter Abschnitt des Aufnahmebereichs anschließen. In diesem dritten Aufnahmebereich kann zur Aufnahme des freien Endes des Steckerkontaktstiftes eine insbesondere zylindrische Aussparung in einem Buchsenteil vorgesehen sein. Das Buchsenteil kann integraler oder separater Bestandteil des Gehäuses sein. Der Durchmesser oder der Querschnitt der Gehäuseöffnung und gegebenenfalls der Aussparung im Buchsenteil sind größer als der Maximalabstand zwischen den beiden Kontaktelementen oder größer als der größte Durchmesser eines einsteckbaren Steckerkontaktstiftes. Die mechanische Klemmung des Steckerkontaktstiftes wird nicht oder lediglich unwesentlich im ersten und gegebenenfalls dritten Abschnitt des Aufnahmebereichs, sondern insbesondere ausschließlich durch die Kontaktelemente im zweiten Abschnitt bewirkt.

Die aufgrund der mechanischen Klemmung durch die Kontaktelemente notwendige Schub- oder Zugkraft zum Bewegen eines Steckerkontaktstiftes oder eines Steckers mit mehreren Steckerkontaktstiften in seiner Erstreckungsrichtung beträgt vorzugsweise zumindest 15 N und maximal 60 N. Vorzugsweise sind die Kontaktelemente in Einsteckrichtung unbeweglich angeordnet und insbesondere weder verschiebbar, noch schwenkbar oder um eine Achse quer zur Einsteckrichtung drehbar. Dadurch kann eine ausreichend große Schub- oder Zugkraft erzeugt werden.
Die Länge des Aufnahmeraums in Einsteckrichtung des Steckerkontaktstiftes beträgt etwa 14 mm bis 16 mm.

Die Buchsenanordnung weist außerdem eine Federeinrichtung auf. Die Federeinrichtung beaufschlagt das zweite Kontaktelement mit einer Vorspannkraft in Richtung zum ersten Kontaktelement hin. Die Vorspannkraft ist insbesondere ausschließlich in eine Richtung radial zur Einsteckrichtung orientiert. Bevorzugt ist die Federeinrichtung gegenüber den beiden Kontaktelementen der Steckerbuchse elektrisch isoliert. Die Federeinrichtung bewirkt, dass ein zwischen die beiden Kontaktelemente eingesteckter Steckerkontaktstift sowohl von entgegengesetzten Seiten her mechanisch beaufschlagt, als auch elektrisch mit dem jeweiligen Kontaktelement verbunden ist. Dadurch, dass die beiden Kontaktelemente relativ zueinander beweglich gelagert sind, können Steckerkontaktstifte mit unterschiedlicher. Durchmessern mechanisch und elektrisch sicher in der Steckerbuchse gehalten bzw. kontaktiert werden. Die Kontaktelemente selbst sind bei den hier auftretenden Kräften starr und nicht elastisch verformbar ausgeführt.

Bei eingestecktem Steckerkontaktstift schließt dieser die beiden Kontaktelemente kurz. Diese elektrische Kurzschlussverbindung kann erkannt und/oder ausgewertet werden. Das erste und das zweite Kontaktelement können identisch ausgeführt sein. Vorzugsweise weist das Gehäuseteil für jede Steckerbuchse eine Gehäuseöffnung auf, durch die jeweils ein Steckerkontaktstift einsteckbar ist. Die Kontaktelemente sind insbesondere durch das Gehäuseteil gegen eine versehentliche Berührung gesichert und in Einbaulage im elektromedizinischen Gerät von außen nicht zugänglich.

Bei nicht in die Steckerbuchse eingestecktem Steckerkontaktstift befinden sich die beiden Kontaktelemente in ihrer Ausgangslage. Der Mindestabstand zwischen den beiden Kontaktelementen ist in der Ausgangslage vorzugsweise zumindest so groß, dass sich die Kontaktelemente nicht berühren und ein Steckerkontaktetift einführbar ist. Der Mindestdurchmesser eines in die Steckerbuchse einsteckbaren Steckerkontaktstifts ist durch den Mindestabstand der beiden Kontaktelemente in ihrer Ausgangslage begrenzt. Der Mindestabstand ist beispielsweise kleiner als 3,7 oder 3,8 mm. Der Maximaldurchmesser eines in die Steckerbuchse einsteckbaren Steckerkontaktstifts ist durch den Maximalabstand der beiden Kontaktelemente vorgegeben, der beispielsweise in einem Bereich von 5,0 mm bis 5,5 mm liegen kann. Die beiden Kontaktelemente können zumindest um mehr als 1 mm und insbesondere um zumindest 1,3 mm relativ zueinander bewegt werden.

Dadurch können beispielsweise sowohl radial federelastische Steckerkontaktstifte mit Federlamellen und starre Steckerkontaktstifte ohne Federlamellen in der Steckerbuchse aufgenommen werden. Für alle Steckerarten und Durchmesser wird eine ausreichende mechanische Klemmwirkung erzeugt.

Bei einer bevorzugten Ausführungsform sind beide Kontaktelemente einer gemeinsamen Steckerbuchse relativ zu dem Gehäuseteil beweglich gelagert. Alternativ hierzu kann auch lediglich das zweite Kontaktelement relativ zum Gehäuseteil beweglich angeordnet sein. Abhängig von der Ausführung wird die beim Einstecken eines Steckerkontaktstifts erzeugte Auslenkung zwischen den beiden Kontaktelementen durch die Bewegung des zweiten Kontaktelements oder durch die Bewegung beider Kontaktelemente relativ zum Gehäuse bewirkt.

Insbesondere weisen die beiden Kontaktelemente einer gemeinsamen Steckerbuchse unterschiedliche elektrische Potenziale auf. Dieser Potenzialunterschied kann durch einen Isolator oder anderes Potenzialunterschiede bewirkendes Mittel bewirkt werden. Beispielsweise sind die beiden Kontaktelemente elektrisch voneinander isoliert oder deren Potenziale voneinander verschieden, solange kein Steckerkontaktstift in die Steckerbuchse eingesteckt ist. Bei dieser Ausgestaltung kann ein eingesteckter Steckerkontakt z.B. durch das Bewerten der Potenziale und/oder der elektrischen Verbindung zwischen den beiden Kontaktelementen sehr einfach erkannt werden.

Die Buchsenanordnung kann bei einem Ausführungsbeispiel eine Steckererkennungseinrichtung aufweisen. Diese Steckererkennungseinrichtung ist mit einer Datenschnittstelle der Buchsenanordnung und/oder mit den beiden Kontaktelementen der Steckerbuchse elektrisch verbunden. Über die Datenschnittstelle können an das Gerät Informationen über den angeschlossenen Instrumententyp oder Statusinformationen über den Betrieb des Instruments übermittelt werden. Zusätzlich oder alternativ hierzu kann die elektrische Verbindung zwischen den beiden Kontaktelementen erkannt bzw. ausgewertet werden, um einen eingesteckten Steckerkontaktstift festzustellen. Bei einer Buchsenanordnung mit mehreren Steckerbuchsen kann abhängig davon, in welcher Steckerbuchse ein Steckerkontaktstift eingesteckt ist, der angeschlossene Instrumententyp erkannt werden. Beispielsweise kann die Steckererkennungseinrichtung monopolare und bipolare elektromedizinische, chirurgische Instrumente unterscheiden. Zudem kann über die Steckererkennungseinrichtung bei ordnungsgemäßer Kontaktierung ein Freigabesignal erzeugt werden, das dem Gerät signalisiert, dass eine Hochfrequenzspannung an das oder die betreffenden Kontaktelemente einer Steckerbuchse angelegt werden kann.

Erfindungsgemäß ist das erste Kontaktelement auf einem elektrisch nicht leitfähigen ersten Kontaktträger und/oder das zweite Kontaktelement auf einem elektrisch nicht leitfähigen zweiten Kontaktträger angeordnet. Der Kontaktträger kann beispielsweise aus Kunststoff hergestellt sein. Beispielsweise ist das Kontaktelement ring- oder hülsenförmig gestaltet und auf dem zugeordneten Kontaktträger drehbar oder unbeweglich gelagert. Die Kontaktelemente können auch eine quaderähnliche Kontur mit einem dem Aufnahmebereich zugewandten in Umfangsrichtung um die Einsteckrichtung gekrümmt verlaufenden Kontaktflächenbereich aufweisen. Die Kontaktelemente können aus Messing, Kupfer, Kupferberyllium oder einem anderen elektrisch leitfähigen Material, insbesondere einem Metall oder einer Metalllegierung, hergestellt sein. Die Kontaktelemente können veredelt, insbesondere vernickelt und/oder vergoldet sein. Über den elektrisch isolierenden Kontaktträger kann sehr einfach eine elektrisch isolierende Lagerung des betreffenden Kontaktelements erfolgen. Die Größe und Anzahl der elektrisch leitenden Bauteile kann minimiert werden. Dadurch lässt sich die Gefahr reduzieren, dass elektrisch leitende Bauteile beim Betrieb des Geräts wie eine Antenne wirken und eine unerwünschte elektromagnetische Strahlung aussenden.

Die Beweglichkeit der beiden Kontaktelement relativ zueinander kann sehr einfach dadurch erreicht werden, dass der erste Kontaktträger und/oder der zweite Kontaktträger beweglich am Gehäuseteil gelagert ist. Die Relativbeweglichkeit kann über eine Führungseinrichtung in eine Bewegungsrichtung beschränkt werden, so dass als Relativbewegung beispielsweise eine Linearbewegung vorgegeben wird. Die Bewegungsrichtung ist vorzugsweise radial zur Einsteckrichtung orientiert. Bei einem Ausführungsbeispiel bilden die beiden Kontaktträger gemeinsam eine Führungseinrichtung, so dass sie unmittelbar aneinander in Bewegungsrichtung verschiebbar gelagert sind. Bei dieser Ausführung können die Kontaktträger mit den Kontaktelementen und optional zusätzlich mit der Federeinrichtung eine Baueinheit bilden, die vormontierbar und als Modul in die Buchsenanordnung einsetzbar ist.

Es besteht auch die Möglichkeit, dass der zweite Kontaktträger und/oder der erste Kontaktträger gemeinsam mit dem Gehäuseteil eine Führungseinrichtung bildet. Beispielsweise kann der erste Kontaktträger fest am Gehäuseteil angeordnet sein, während der zweite Kontaktträger über eine Führungseinrichtung bewegbar am Gehäuseteil gelagert ist. Bei dieser Ausführung kann die Anzahl der bewegbar gelagerten Teile reduziert werden.

Bei einem bevorzugten Ausführungebeispiel beaufschlagt die Federeinrichtung zumindest einen der Kontaktträger. Eine unmittelbare mechanische und/oder elektrische Verbindung zwischen der Federeinrichtung und einem Kontaktelement kann auf diese Weise vermieden werden. Über den isolierenden Kontaktträger kann die Federeinrichtung elektrisch getrennt werden von dem jeweils an den Kontaktträger angeordneten Kontaktelement.

Es ist möglich, dass sich die Federeinrichtung am ersten Kontaktträger und/oder am zweiten Kontaktträger abstützt. Dabei kann die Federeinrichtung auf Zug belastet sein. Es ist ferner möglich, dass sich die Federeinrichtung einerseits unmittelbar am ersten Kontaktelement oder am Gehäuseteil und andererseits am zweiten Kontaktträger abstützt. Bei dieser Variante kann die Federeinrichtung vorzugsweise auf Druck belastet sein.

Als Federeinrichtung dient bei allen Ausführungen wenigstens ein Federelement, das eine Blattfeder, eine Schraubenfeder, ein elastisch verformbares Federelement, beispielsweise ein Elastomerring, sein kann. Die Federeinrichtung kann mehrere der genannten Federelemente gleichen Typs oder unterschiedlichen Typs aufweisen. Das wenigstens eine Federelement einer Federeinrichtung ist vorzugsweise elektrisch nicht leitfähig.

Wenn die Buchsenanordnung wenigstens zwei Steckerbuchsen aufweist, können die beiden ersten Kontaktelemente und die beiden zweiten Kontaktelemente jeweils auf einem gemeinsamen Kontaktträger gelagert sein. Dadurch kann die Anzahl der Bauteile reduziert werden. Es ist ferner möglich, dass die beiden Steckerbuchsen eine gemeinsame Federeinrichtung aufweisen. Die auf einem gemeinsamen Kontaktträger angeordneten ersten oder zweiten Kontaktelemente können elektrisch gegeneinander isoliert sein.

Um das Einstecken unterschiedlicher Stecker mit verschiedenen Kontaktstiftabständen zu erleichtern, kann wenigstens eine Steckerbuchse als Doppelbuchse ausgeführt sein. Diese weist zwei nebeneinander angeordnete Aufnahmebereiche für einen Steckerkontaktstift auf. Die Doppelbuchse kann eine gemeinsame Gehäuseöffnung im Gehäuseteil aufweisen. Beiden Aufnahmebereichen ist vorzugsweise ein gemeinsames erstes und/oder ein gemeinsames zweites Kontaktelement zugeordnet, das mit einem Steckerkontaktstift in mechanischen und elektrischen Kontakt gelangt, unabhängig davon, ob dieser in den einen oder anderen Aufnahmebereich der Doppelbuchse eingesteckt ist. Alternativ hierzu können auch zwei erste und zweite Kontaktelemente vorgesehen sein, die vorzugsweise mechanisch und/oder elektrisch miteinander verbunden sind. Die Flexibilität der Buchsenanordnung wird durch eine solche Doppelbuchse weiter vergrößert und die Anzahl der anschließbaren elektromedizinischen Instrumente erhöht.

Der Kontaktträger für das wenigstens eine erste Kontaktelement und/oder der Kontaktträger für das wenigstens eine zweite Kontaktelement können sich quer zur Einsteckrichtung und bei einer Ausführungsform parallel zueinander erstrecken.

Das erste Kontaktelement und/oder das zweite Kontaktelement einer Steckerbuchse können quer zur Einsteckrichtung in Umfangsrichtung um die Einsteckrichtung konkav gewölbt sein. Dadurch ist eine verbesserte Führung des Steckerkontaktstifts beim Einstecken erreicht. Außerdem kann durch die Wölbung die Kontaktfläche zwischen dem Kontaktelement und dem Steckerkontaktstift vergrößert werden. Vorzugsweise ist zumindest eine linienhafte und insbesondere eine flächige Anlage zwischen jedem Kontaktelement und einem eingesteckten Steckerkontaktstift vorhanden.

Das erste und/oder das zweite Kontaktelement können um eine quer zur Einsteckrichtung und vorzugsweise quer zur Bewegungsrichtung verlaufenden Achse drehbar gelagert sein, beispielsweise auf dem zugeordneten Kontaktträger. Die Lagerung kann über ein Gleitlager oder ein Wälzlager, beispielsweise ein Kugellager, erfolgen.

Weitere vorteilhafte Ausgestaltungen der Erfindung ergeben sich aus den abhängigen Patentansprüchen sowie der Beschreibung. Die Beschreibung beschränkt sich auf wesentliche Merkmale der Erfindung und vorteilhafte Ausführungsbeispiele. Die Zeichnung ist ergänzend heranzuziehen. Nachfolgend wird die Erfindung anhand von Ausführungsbeispielen näher erläutert. Es zeigen:
Figur 1 eine perspektivische Darstellung einer Buchsenanordnung eines elektxomedizinischen Geräts mit Blick auf die Anschlussseite des Buchsengehäuses,
Figur 2 die Buchsenanordnung nach Figur 1 in Draufsicht auf das Buchsengehäuse,
Figuren 3 und 4 jeweils ein Ausführungsbeispiel eines Steckers eines elektromedizinischen, chirurgischen Instruments,
Figur 5 eine perspektivische Darstellung eines ersten Ausführungsbeispiels von Kontaktanordnungen einer Buchsenanordnung,
Figur 6 eine perspektivische Darstellung eines weiteren, zweiten Ausführungsbeispiels von Kontaktanordnungen einer Buchsenanordnung,
Figur 7 eine perspektivische Darstellung eines dritten Ausführungsbeispiels von Kontaktanordnungen einer Buchsenanordnung und
Figuren 8 und 9 je eine schematische Schnittdarstellung durch eine Steckerbuchse einer Buchsenanordnung ohne und mit eingestecktem Steckerkontaktstift.

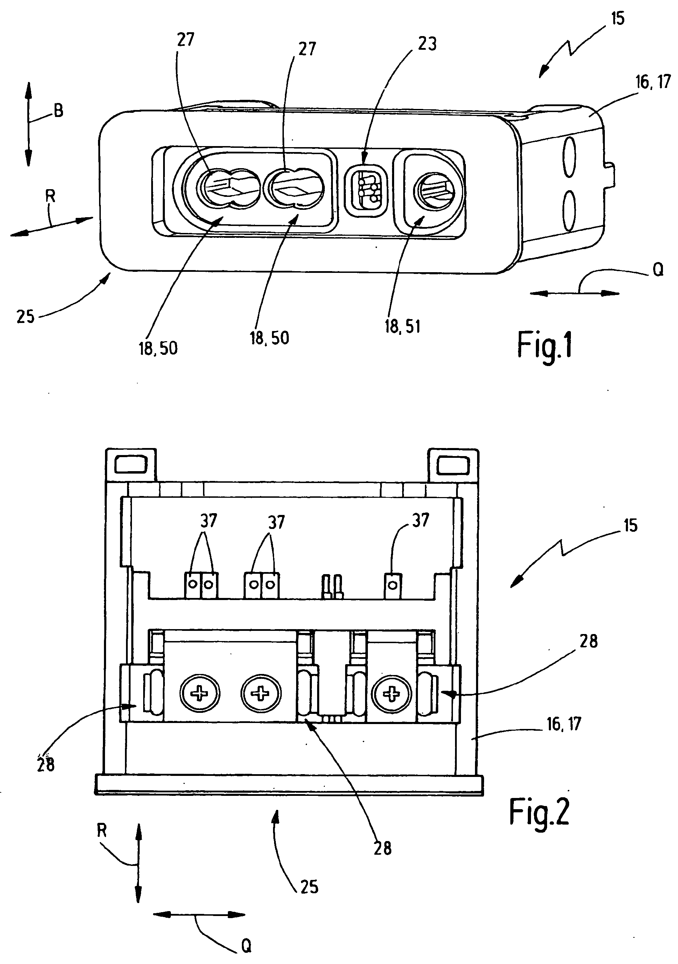

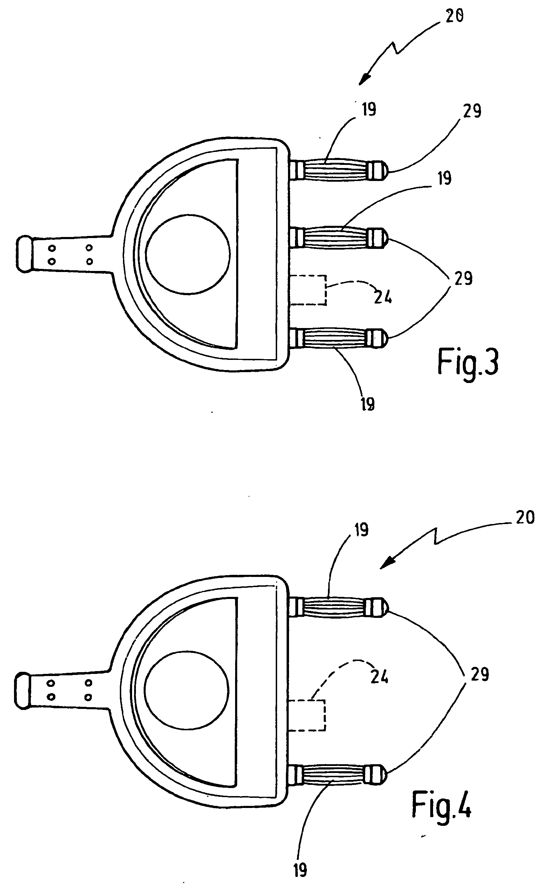

Eine Buchsenanordnung 15 für ein elektromedizinisches Gerät ist in den Figuren 1 und 2 veranschaulicht. Die Buchsenanordnung 15 weist mehrere in einem Gehäuseteil 16, beispielsweise einem Buchsengehäuse 17 angeordnete Steckerbuchsen 18 auf. Das Gehäuseteil besteht aus elektrisch isolierendem Material, insbesondere aus Kunststoff. Jede Steckerbuchse 18 dient zur mechanischen und elektrischen Kontaktierung mit einem Steckerkontaktstift 19 eines Steckers 20 (Figuren 3 und 4). Der Stecker 20 gehört zu einem elektromedizinischen und vorzugsweise chirurgischen Instrument. Der Stecker 20 für ein monopolares chirurgisches Instrument weist drei Steckerkontaktstifte 19 auf (Figur 3), während der Stecker 20 für ein bipolares Instrument zwei Steckerkontaktstifte 19 hat (Figur 4). Die Länge, die Dicke sowie der genaue Abstand der Steckerkontaktstifte 19 eines Steckers 20 sind nicht genau vorgeschrieben, wodurch es bei verschiedenen Herstellern zu Abweichungen kommt. Die Buchsenanordnung 15 ist so ausgeführt, dass Stecker 20 unterschiedlichen Typs und mit voneinander abweichenden Dimensionierungen und Abständen der Steckerkontaktstifte 19 mechanisch und elektrisch anschließbar sind.

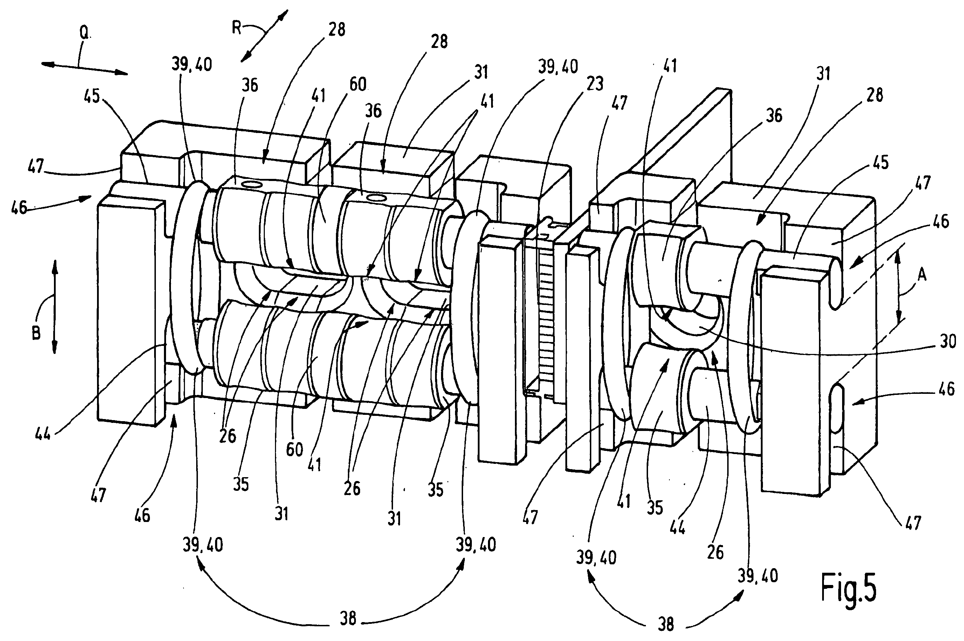

Zu der Buchsenanordnung 15 kann neben den Steckerbuchsen 18 auch eine Datenschnittstelle 23 gehören. Am Stecker 20 kann passend zur Datenschnittstelle 23 ein Steckkontakt 24 vorhanden sein. Das Instrument kann über den Stecker 20 und den Steckkontakt 24 Informationen an das elektromedizinische Gerät übertragen, beispielsweise den aktuellen Betriebszustand des Instruments, Fehlerzustände, Informationen über den Instrumententyp etc. Beim Ausführungsbeispiel nach Figur 1 ist die Datenschnittstelle 23 als vierpoliger Kontakt ausgeführt. Auch andere Formen von Datenschnittstellen 23, wie etwa USB- oder Mini-USB-Schnittstellen oder Micro-SD-Schnittstellen (Figur 5) können verwendet werden. Vorzugsweise weist die Datenschnittstelle 23 zumindest vier Pole auf. Die Anschlüsse oder Kontakte der Datenschnittstelle 23 sind auf der Anschlussseite 25 des Buchsengehäuses 17 bzw. des Gehäuseteils 16 hinter einer Ebene angeordnet, die durch die Anschlussseite 25 des Gehäuseteils 16 definiert ist. Dadurch wird das Einstecken eines Steckers 20 nicht behindert, der keine Anschlussmöglichkeit für die Datenschnittstelle 23 aufweist.

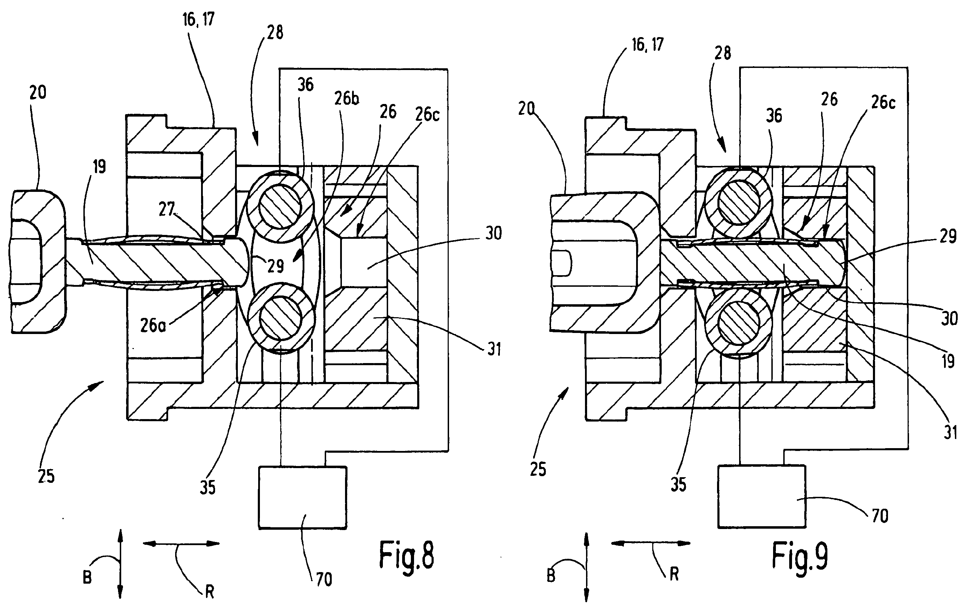

Jede Steckerbuchse 18 weist einen Aufnahmebereich 26 für den zugeordneten Steckerkontaktstift 19 auf. Der Aufnahmebereich 26 ist insbesondere in den Figuren 8 und 9 vollständig dargestellt. Der Aufnahmebereich 26 erstreckt sich von der Anschlussseite 25 in einer Einsteckrichtung R, in der der Steckerkontaktstift 19 in die Steckerbuchse 18 eingesteckt wird. Zu dem Aufnahmebereich 26 gehört eine Gehäuseöffnung 27, an der das Gehäuseteil 16 bzw. das Buchsengehäuse 17 zur Anschlussseite 25 hin offen ist. Die Gehäuseöffnung 27 bildet einen ersten Abschnitt 26a des Aufnahmebereichs 26. An die Gehäuseöffnung 27 schließt sich ein zweiter Abschnitt 26b des Aufnahmebereichs 26 an. Der zweite Abschnitt 26b befindet sich im Bereich einer Kontaktanordnung 28, die zur mechanischen Klemmung und zur elektrischen Kontaktierung eines eingesteckten Steckerkontaktstiftes 19 dient. Ein beispielsgemäß vorhandener dritter Abschnitt 26c des Aufnahmebereichs 26 dient zur Aufnahme des freien Endes 29 des Steckerkontaktstiftes 19. Der dritte Abschnitt 26c ist durch eine in Einsteckrichtung R verlaufende Aussparung 30 eines Buchsenteils 31 vorgesehen. Das Buchsenteil kann integraler oder separater Bestandteil des Gehäuseteils 16 und mithin des Buchsengehäuses 17 sein.

Über den ersten Abschnitt 26a in der Gehäuseöffnung 27 und den dritten Abschnitt 26c im Buchsenteil 31 ist ein eingesteckter Steckerkontaktstift 19 in etwa in Einsteckrichtung R orientiert angeordnet. Der Querschnitt oder der Durchmesser der Gehäuseöffnung 27 und der Aussparung 30 im Buchsenteil 31 sind so gewählt, dass alle einsteckbaren Querschnittsformen und Durchmessergrößen der Steckerkontaktstifte 19 aufgenommen werden können. In Abwandlung zu der beispielhaften Darstellung in den Figuren 8 und 9 muss der Außendurchmesser des Steckerkontaktstiftes 19 daher nicht zwingend dem Innendurchmesser der Gehäuseöffnung 27 bzw. der Aussparung 30 entsprechen, sondern kann auch kleiner sein. Mit anderen Worten, sind der Querschnitt beziehungsweise der Durchmesser der Gehäuseöffnung 27 und der Aussparung 30 größer als der Querschnitt beziehungsweise der Durchmesser des Steckerkontaktstiftes, wirkt weder im ersten Abschnitt 26a, noch im dritten Abschnitt 26c des Aufnahmebereichs 26 eine beachtliche Haltekraft auf den Steckerkontaktstift. Die Klemmung des in die Steckerbuchse eingesteckten Steckerkontaktstiftes erfolgt beispielsgemäß ausschließlich im zweiten Abschnitt 26b durch die Kontaktanordnung 28.

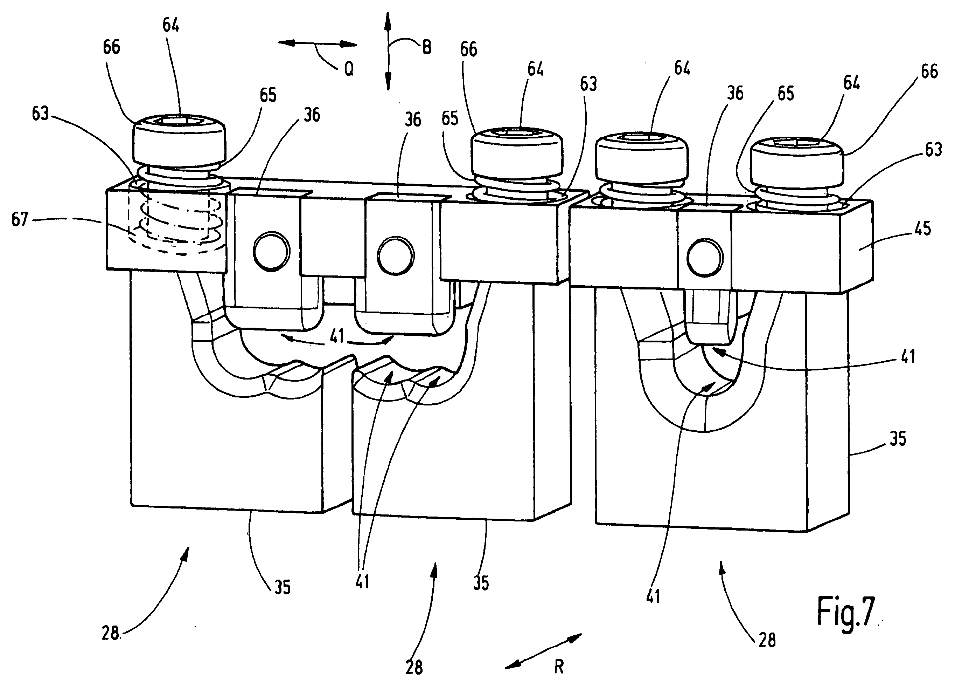

Verschiedene Ausführungsbeispiele der Kontaktanordnung 28 sind in den Figuren 5 bis 7 veranschaulicht. Zur besseren Darstellung der Kontaktanordnung 28 ist die Anschlussseite des Gehäuseteils 16 mit der Gehäuseöffnung 27 weggelassen. In Figur 7 ist lediglich die Kontaktanordnung ohne andere Teile der Buchsenanordnung 15 dargestellt.

Jede Kontaktanordnung 28 einer Steckerbuchse 18 weist ein elektrisch leitfähiges erstes Kontaktelement 35 und ein elektrisch leitfähiges zweites Kontaktelement 36 auf. Bevorzugt sind nur zwei Kontaktelemente 35, 36 vorhanden. Die beiden Kontaktelemente 35, 36 sind bei nicht eingestecktem Steckerkontaktstift 19 elektrisch voneinander getrennt. Beim Ausführungsbeispiel ist eines der beiden Kontaktelemente 35, 36 und vorzugsweise das erste Kontaktelement 35 einer gemeinsamen Steckerbuchse 18 z.B. über eine Anschlussfahne 37 (Figur 2) mit einem entsprechenden Eingang oder Ausgang des elektromedizinischen Gerätes verbunden. Das andere Kontaktelement 35, 36 weist auch eine Anschlussfahne 37 auf, die zur Detektion eines Steckerkontaktstiftes 19 verwendet wird. Wenigstens ein Kontaktelement 35, 36 der Buchsenanordnung 15 ist dazu vorgesehen und eingerichtet, eine Hochfrequenzspannung bzw. einen Hochfrequenzstrom zu übertragen.

Die beiden Kontaktelemente 35, 36 sind mit Abstand zueinander auf gegenüberliegenden Seiten des Aufnahmebereiches 26 und beispielsgemäß des zweiten Abschnitts 26b des Aufnahmebereichs 26 angeordnet. Sie liegen radial zur Einsteckrichtung R in einer Bewegungsrichtung B mit einem Mindestabstand gegenüber. Der Mindestabstand ist vorzugsweise kleiner als 3,7 mm. Das zweite Kontaktelement 36 ist in Bewegungsrichtung B relativ zum ersten Kontaktelement 35 bewegbar. Beim ersten Ausführungsbeispiel der Kontaktanordnung 28 nach Figur 5 sind beide Kontaktelemente 35, 36 einer Steckerbuchse 18 in Bewegungsrichtung B linear verschiebbar am Gehäuseteil 16 bzw. dem Buchsengehäuse 17 gelagert. In Abwandlung hierzu kann auch lediglich das zweite Kontaktelement 36 gegenüber dem Gehäuseteil 16 beweglich gelagert sein. Der Maximalabstand der beiden Kontaktelemente 35, 36, der durch die Relativbewegung der beiden Kontaktelemente 35, 36 erreichbar ist, beträgt beispielsweise zumindest 5,0 mm. Somit sind Steckerkontaktstifte mit unterschiedlichen Durchmessern zumindest im Bereich von 3,7 mm bis 5,0 mm zwischen die beiden Kontaktelemente 35, 36 einsteckbar.

Eine Federeinrichtung 38 dient dazu, eine Vorspannkraft zwischen den beiden Kontaktelementen 35, 36 in Bewegungsrichtung B zu erzeugen. Die Federeinrichtung 38 kann hierfür eine Zugkraft und/oder eine Druckkraft ausüben. Beim bevorzugten Ausführungsbeispiel besteht die Federeinrichtung 38 aus mehreren separaten Federelementen 39. Ein Federelement 39 kann durch eine Schraubenfeder, eine Blattfeder oder ein elastisch verformbares Federelement gebildet sein. Beim Ausführungsbeispiel nach Figur 5 weist jede Federeinrichtung zwei Elastomerringe 40 auf, die jeweils ein Federelement 39 darstellen.

Das erste Kontaktelement 35 sitzt beim ersten Ausführungsbeispiel nach Figur 5 auf einem ersten Kontaktträger 44 und das zweite Kontaktelement 36 sitzt auf einem zweiten Kontaktträger 45. Die Kontaktträger 44, 45 sind aus elektrisch nicht-leitfähigem Kunststoff. Die beiden Kontaktträger 44, 45 sind stabförmig ausgeführt und erstrecken sich in eine Querrichtung Q. Die Querrichtung Q ist rechtwinklig zur Einsteckrichtung R und rechtwinklig zur Bewegungsrichtung B orientiert. Die Elastomerringe 40 der Federeinrichtung 38 sind in Querrichtung Q gesehen auf beiden Seiten des ersten Kontaktelements 35 sowie des zweiten Kontaktelements 36 angeordnet. Die Elastomerringe 40 umschließen die beiden Kontaktträger 44, 45. Sie beaufschlagen daher beide Kontaktträger 44, 45 und ziehen diese mit der Vorspannkraft in Bewegungsrichtung B aufeinander zu.

Erfindungsgemäß sind die Kontaktträger 44, 45 der Kontaktanordnung 28 über jeweils eine Führungseinrichtung 46 in Bewegungsrichtung B verschiebbar geführt am Gehäuseteil 16 bzw. dem Buchsengehäuse 17 gelagert. Hierfür weist die Führungseinrichtung 46 einen in Bewegungsrichtung B verlaufenden Schlitz 47 auf. Anstelle eines Schlitzes 47 kann auch eine nutähnliche Aussparung im Gehäuseteil 16 vorhanden sein. Der zugeordnete Kontaktträger 44, 45 greift in den Schlitz 47 ein. Er ist in Bewegungsrichtung B verschiebbar gelagert. Zur Verdrehsicherung des betreffenden Kontaktträgers 44, 45 kann dieser abweichend von seiner ansonsten vorzugsweise zylindrischen Form zwei Abflachungen aufweisen, mit denen er an den Schlitzwänden des Schlitzes 47 gleitend anliegt.

Jeder der Kontaktträger 44, 45 ist beispielsgemäß mit seinen beiden Endabschnitten in jeweils einem Schlitz 47 des Gehäuseteils 16 gelagert. Über den in Bewegungsrichtung B gemessenen Abstand A zwischen den Schlitzen 47 für den ersten Kontaktträger 44 und den Schlitzen 47 für den zweiten Kontaktträger 45 einer Kontaktanordnung 28 kann der Abstand der beiden Kontaktelemente 35, 36 in deren Ausgangslage bei nicht eingestecktem Steckerkontaktstift 19 vorgegeben werden.

Beim ersten Ausführungsbeispiel der Kontaktanordnung 28 nach Figur 5 sind das erste Kontaktelement 35 und das zweite Kontaktelement 36 auf dem jeweils zugeordneten Kontaktträger 44, 45 unbeweglich angeordnet. In Abwandlung hierzu kann ein Kontaktelement oder beide Kontaktelemente 35, 36 um eine sich in Querrichtung Q erstreckende Drehachse drehbar auf dem Kontaktträger 44 bzw. 45 gelagert sein. Die Drehlagerung kann mit Hilfe eines Gleit- oder Wälzlagers erfolgen. Dadurch kann die Reibung zwischen den Kontaktelementen und dem Steckerkontaktstift bei dessen Bewegung in Einsteckrichtung R in den Aufnahmebereich 26 bzw. aus dem Aufnahmebereich 26 heraus reduziert werden.

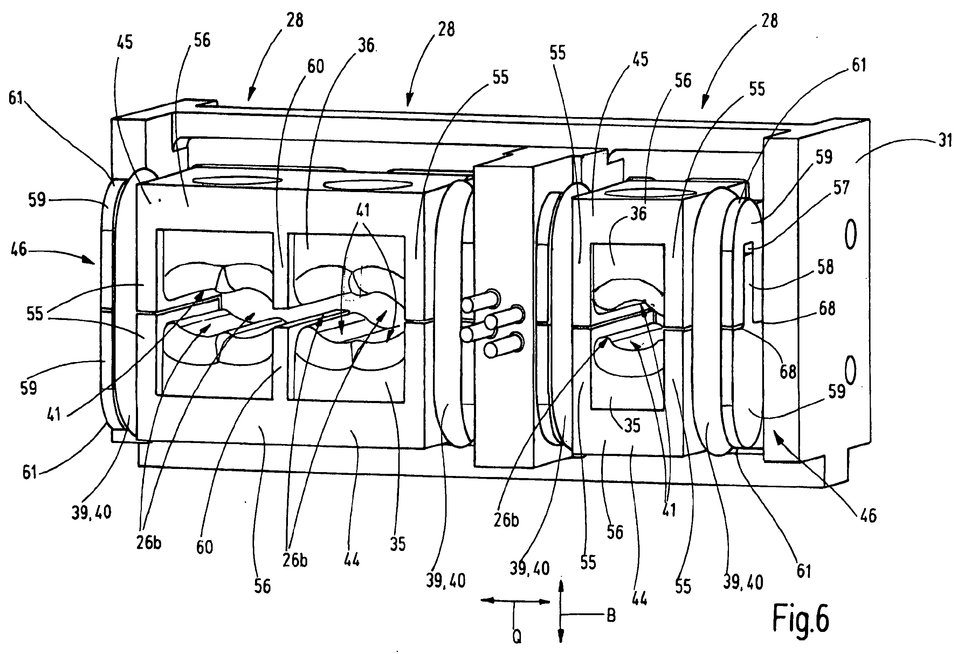

Die Kontaktelemente 35, 36 weisen jeweils einen an den Aufnahmeraum 26 angrenzenden Kontaktflächenbereich 41 auf. Wie in Figur 5 veranschaulicht, kann der Kontaktflächenbereich 41 der Kontaktelemente 35, 36 in Querrichtung Q konkav gewölbt ausgeführt sein. Durch diese Wölbung kann in Querrichtung Q eine gewisse Führung oder Zentrierung des Steckerkontaktstiftes 19 erreicht werden. In Abwandlung zu der in Figur 5 dargestellten Ausführung kann auch nur eines der beiden Kontaktelemente 35, 36 mit einer entsprechenden Wölbung des Kontaktflächenbereichs 41 versehen sein, wohingegen das jeweils andere Kontaktelement eine in Querrichtung Q gerade verlaufende Außenfläche aufweist. Wegen der Hülsenform oder hohlzylinderartigen Form der Kontaktelemente 35, 36 bei dem ersten Ausführungsbeispiel der Kontaktanordnung 28 sind die Kontaktflächenbereiche 41 in Einsteckrichtung konvex gewölbt. Es ist zusätzlich oder alternativ auch möglich, Kontaktelemente 35, 36 mit in Einsteckrichtung gerade verlaufenden Kontaktflächenbereichen 41 vorzusehen, wie dies bei den Ausführungsformen der Figuren 6 und 7 veranschaulicht ist.

Die Kontaktelemente 35, 36 können eine zylindrische Durchgangsöffnung aufweisen, mit der sie auf den jeweiligen Kontaktträger 44 bzw. 45 aufgesteckt werden können. Die Kontaktelemente 35, 36 sind aus elektrisch leitfähigem Material, beispielsweise Metall oder einer Metalllegierung, die auch veredelt, beispielsweise vergoldet oder vernickelt sein kann. Die Kontaktelemente 35, 36 können lösbar oder unlösbar mit dem jeweiligen Kontaktträger 44, 45 verbunden sein. Es ist auch möglich, den Kontaktträger 44, 45 an das Kontaktelement 35 bzw. 36 anzuformen, beispielsweise durch Spritzgießen.

Die Buchsenanordnung 15 weist bei den hier beschriebenen Ausführungsformen drei Steckerbuchsen 18 auf, wobei eine oder mehrere Steckerbuchsen 18 als Doppelbuchsen 50 mit jeweils zwei Aufnahmebereichen 26 für einen Steckerkontaktstift 19 ausgeführt sind. Beispielsgemäß ist eine einzige Steckerbuchse 18 als Einzelbuchse 51 mit lediglich einem Aufnahmebereich 26 für einen Steckerkontaktstift 19 ausgeführt. Die Einzelbuchse 51 dient insbesondere als Referenzbuchse, die unabhängig vom Instrumententyp oder der Steckerbauform bei einem eingesteckten Stecker 20 immer mit einem Steckerkontaktstift 19 belegt ist. Die Datenschnittstelle 23 ist vorzugsweise zwischen der Einzelbuchse 51 und den beispielsgemäß zwei Doppelbuchsen 50 angeordnet.

Die Kontaktanordnung 28 für eine Doppelbuchse 50 ist im Wesentlichen entsprechend der Kontaktanordnung 28 für eine Einzelbuchse 51 aufgebaut. Jede Kontaktanordnung 28 verfügt jeweils über ein erstes Kontaktelement 35 auf einem ersten Kontaktträger 44, ein zweites Kontaktelement 36 auf einem zweiten Kontaktträger 45 sowie eine Federeinrichtung 38 zur Erzeugung einer Vorspannkraft zwischen den beiden Kontaktträgern 44, 45 bzw. den beiden Kontaktelementen 35, 36. Bei der als Doppelbuchse 50 ausgeführten Steckerbuchse 18 ist für beide Aufnahmebereiche 26 ein gemeinsames erstes Kontaktelement 35 und ein gemeinsames zweites Kontaktelemente 36 vorgesehen. Die Form der Kontaktelemente 35, 36 einer Doppelbuchse 50 entspricht im Wesentlichen der Form von zwei unmittelbar in Querrichtung Q aneinander anschließenden Kontaktelementen 35, 36 der Einzelbuchse 51. Die Kontaktelemente 35, 36 der Doppelbuchse 50 weisen mithin zwei in Querrichtung Q aneinander anschließende konkav gewölbte Kontaktflächenbereiche 41 auf. Alternativ könnten auch jeweils zwei separate erste und zweite Kontaktelemente für jeden Aufnahmebereich 26 der Doppelbuchse 50 vorgesehen sein, die insbesondere elektrisch und/oder mechanisch unmittelbar miteinander verbunden sein können.

Bei dem in Figur 5 dargestellten ersten Ausführungsbeispiel weisen die beiden Kontaktanordnungen 28 für die beiden Doppelbuchsen 50 einen gemeinsamen ersten Kontaktträger 44 und/oder einen gemeinsamen zweiten Kontaktträger 45 auf. Die den beiden Kontaktträgern 44, 45 zugeordnete Federeinrichtung 38 ist ebenfalls beiden Doppelbuchsen 50 zugeordnet. Die beiden zu unterschiedlichen Steckerbuchsen 18 bzw. Doppelbuchsen 50 gehörenden und auf einem gemeinsamen Kontaktträger 44 oder 45 angeordneten ersten Kontaktelemente 35 bzw. zweiten Kontaktelemente 36 sind über einen Isolatorkörper 60 elektrisch voneinander isoliert. Der Isolatorkörper 60 ist beispielsgemäß als Ringkörper ausgeführt und kann integraler Bestandteil des betreffenden Kontaktträgers 44 oder 45 sein.

Allgemein ausgedrückt können Kontaktanordnungen 28 benachbarter Steckerbuchsen 18 bei allen Ausführungsformen gemeinsame Bauteile aufweisen, insbesondere einen gemeinsamen ersten Kontaktträger 44 und/oder einen gemeinsamen zweiten Kontaktträger 45 und/oder eine gemeinsame Federeinrichtung 38.

Wie im Zusammenhang mit der Kontaktanordnung 28 der Einzelbuchse 51 beschrieben, bilden auch die Kontaktträger 44, 45 mit dem Gehäuseteil 16 jeweils eine Führungseinrichtung 46, wobei die axialen Endabschnitte der Kontaktträger 44, 45 in Schlitzen 47 verschiebbar geführt gelagert sind. Die Federeinrichtung 38 ist für die Kontaktanordnungen 28 der beiden Doppelbuchsen 50 durch zwei als Elastomerringe 40 ausgeführte Federelemente 39 gebildet, wie dies vorstehend bereits geschildert wurde.

Durch das Vorsehen von zwei Doppelbuchsen 50 mit jeweils zwei in Querrichtung Q nebeneinander liegenden Aufnahmebereichen 26 für einen Steckerkontaktstift 19 können unterschiedliche Abstände von Steckerkontaktstiften 19 bei Steckern 20 toleriert werden. Abhängig vom Abstand zweier Steckerkontaktstifte 19 greift dieser in den einen oder den anderen Aufnahmebereich 26 einer Doppelbuchse 50 ein. Bei Steckern 20 von monopolaren Instrumenten sind alle Steckerbuchsen 18 durch jeweils einen Steckerkontaktstift 19 belegt. Bei bipolaren Instrumenten weist der Stecker 20 nur zwei Steckerkontaktstifte 19 auf, von denen einer der Einzelbuchse 51 und der andere einer der beiden Doppelbuchsen 50 zugeordnet ist.

Wie Figur 5 außerdem veranschaulicht, ist die Aussparung 30 im Buchsenteil 31 bei der Einzelbuchse 51 in etwa zylindrisch und bei der Doppelbuchse 50 in Form eines sich in Querrichtung Q erstreckenden Langloches für die beiden nebeneinander liegenden Aufnahmebereiche 26 ausgestaltet.

Figur 6 zeigt ein abgewandeltes, zweites Ausführungsbeispiel der Kontaktanordnung 28 für eine Steckerbuchse 18 einer Buchsenanordnung 15. Gleiche Bauteile der Kontaktanordnung 28 bzw. der Buchsenanordnung 15 sind mit denselben Bezugszeichen versehen. Im Folgenden werden lediglich die Unterschiede gegenüber der bisher beschriebenen ersten Ausführungsform erläutert.

Der Hauptunterschied der Ausführungsform der Kontaktanordnungen 28 gemäß Figur 6 besteht in der Form und Ausführung der Kontaktträger 44, 45 sowie der Kontaktelemente 35, 36. Im Unterschied zu der bisher beschriebenen Variante sind die beiden Kontaktträger 44, 45 bügel- oder U-förmig mit zwei in Bewegungsrichtung B verlaufenden und in Querrichtung Q beabstandeten Schenkeln 55 und einem die beiden Schenkel 55 in Querrichtung Q verbindenden Querstück 56 ausgeführt. Die Schenkel 55 und das Querstück 56 können quaderförmig sein. Zwischen den beiden Schenkeln 55 ist jeweils das betreffende erste Kontaktelement 35 bzw. das zweite Kontaktelement 36 befestigt. Die Kontaktelemente 35, 36 liegen auf drei Seiten an den beiden Schenkeln 55 und am Querstück 56 vorzugsweise flächig an. Auf ihrer dem Aufnahmeraum 26 und insbesondere dem zweiten Abschnitt 26b des Aufnahmeraums zugewandten Seite sind die Kontaktflächenbereiche 41 der Kontaktelemente 35, 36 wie beim ersten Ausführungsbeispiel in Querrichtung Q konkav gewölbt. Die ersten und zweiten Kontaktelemente 35, 36, die zu den Doppelbuchsen 50 gehören, weisen jeweils zwei benachbarte konkav gewölbte Kontaktflächenbereiche 41 auf. Bei dem in Figur 6 dargestellten zweiten Ausführungsbeispiel sind die Kontaktflächenbereiche 41 in Einsteckrichtung R gerade. Dadurch kann eine größere Kontaktfläche mit dem Steckerkontaktstift 19 erreicht werden.

Ein weiterer Unterschied gegenüber der ersten Ausführungsform besteht bei der zweiten Ausführungsform darin, dass die beiden zu einer Steckerbuchse 18 gehörenden Kontaktträger 44, 45 unmittelbar aneinander in Bewegungsrichtung B verschiebbar geführt gelagert sind. Die Führungseinrichtung 46 ist durch die beiden Kontaktträger 44, 45 und gebildet. Beispielsgemäß weist der zweite Kontaktträger 45 an beiden Schenkeln 55 jeweils eine Führungsaussparung 57 auf, die sich in Bewegungsrichtung B erstreckt. Die Führungsaussparung 57 kann von einer Nut oder einem Schlitz gebildet sein. In dieser Führungsaussparung 57 greift ein in Bewegungsrichtung B vom ersten Führungsträger 44 wegragender Führungsvorsprung 58 ein. Die Länge der Führungsaussparung 57 und des Führungsvorsprungs 58 sind an die Auslenkung der beiden Kontaktträger 44, 45 bei ihrer Relativbewegung in Bewegungsrichtung B angepasst. Der Führungsvorsprung 58 bleibt in jeder Relativposition im Eingriff mit der Führungsaussparung 57. Es versteht sich, dass die Führungsaussparung alternativ auch am ersten Kontaktträger 44 und der Führungsvorsprung 58 am zweiten Kontaktträger 45 vorhanden sein könnte. Die Kontaktträger 44, 45 weisen Positionsmittel auf (nicht dargestellt), die mit entsprechenden komplementären Positionsmitteln (nicht dargestellt) am Buchsenteil 31 bzw. am Buchsengehäuse 17 zusammenwirken. Dadurch wird die Positionierung der Kontaktträger 44, 45 in R- und Q-Richtung sichergestellt.

Der Führungsvorsprung 58 bzw. die Führungsaussparung 57 sind jeweils an einem betreffenden Querfortsatz 59 des Schenkels 55 vorhanden. Die beiden Kontaktträger 44, 45 und insbesondere die beiden Querfortsätze 59 einer gemeinsamen Kontaktanordnung 28 liegen in der Ausgangslage durch die Vorspannkraft der Federeinrichtung 38 in Bewegungsrichtung B mit Anschlagflächen 68 aneinander an. An ihren in Bewegungsrichtung B entgegengesetzten Seiten sind einander zugeordnete Querfortsätze 59 des ersten Kontaktträgers 44 und des zweiten Kontaktträgers 45 mit abgerundeten vorzugsweise kreisbogenförmigen Außenflächenbereichen 61 versehen, um die die Elastomerringe 40 herum verlaufen. In der Ausgangslage bilden zwei unmittelbar benachbarte Querfortsätze 59 der beiden Kontaktträger 44, 45 einer Kontaktanordnung 28 mit Blickrichtung in Querrichtung Q eine in etwa rechteckige Kontur, deren Endbereiche gerundet ausgebildet sind.

Wie beim zuerst beschriebenen Ausführungsbeispiel weisen die beiden Kontaktanordnungen 28 der beiden Doppelbuchsen 50 einen gemeinsamen ersten Kontaktträger 44 und einen gemeinsamen zweiten Kontaktträger 45 auf, der jedenfalls jeweils zwei Schenkel 55 mit einem Querstück 56 aufweist. Zwischen den beiden ersten Kontaktelementen 35 und den beiden zweiten Kontaktelementen 36 ist jeweils ein Isolator 60 zur elektrischen Isolation der auf einem gemeinsamen Kontaktträger 44, 45 angeordneten Kontaktelemente 35 bzw. 36 vorhanden. Der Isolator 60 ist beispielsgemäß quaderförmig und parallel zu den beiden Schenkeln ausgerichtet. Er kann integraler Bestandteil des jeweiligen Kontaktträgers 44, 45 sein.

Bei dem bisher beschriebenen ersten und zweiten Ausführungsbeispiel der Kontaktanordnung 28 sind jeweils beide Kontaktträger 44, 45 und mithin auch beide Kontaktelemente 35, 36 einer Kontaktanordnung 28 in Bewegungsrichtung B bewegbar gegenüber dem Gehäuseteil 16 gelagert. Die dritte Ausführungsform nach Figur 7 weicht hiervon ab. Die ersten Kontaktelemente 35 jeder Kontaktanordnung 28 sind hier unbeweglich gegenüber dem Gehäuseteil 16 bzw. dem Buchsengehäuse 17 gelagert. Wie beim Ausführungsbeispiel nach Figur 6 weisen die ersten Kontaktelemente 35 angrenzend an jeden Aufnahmebereich 26 einen jeweils in Querrichtung Q konkav gewölbten Kontaktflächenabschnitt 41 auf. Der Kontaktträger für die ersten Kontaktelemente 35 ist durch einen nicht dargestellten Halteabschnitt des Gehäuseteils 16 bzw. des Buchsengehäuses 17 gebildet.

Lediglich das zweite Kontaktelement 36 ist über den zweiten Kontaktträger 45 in Bewegungsrichtung B relativ zum Gehäuseteil 16 linear verschiebbar gelagert. Der zweite Kontaktträger 45 ist quaderförmig gestaltet und weist zwei in Bewegungsrichtung B verlaufende Durchgangsöffnungen 63 auf. Durch diese Durchgangsöffnungen 63 ragt ein Stift 64, beispielweise eine Schraube, wobei der Stift 64 unmittelbar mit dem zugeordneten ersten Kontaktelement 35 verbunden ist. Der Stift kann bei einer Ausführung gegenüber dem ersten Kontaktelement 35 isoliert sein oder aus elektrisch nicht leitfähigem Material bestehen. Alternativ kann der Stift 64 aus leitfähigem Material gebildet und mit dem Kontaktelement 35 leitfähig verbunden sein. Dies erfordert dann, dass der Stift 64 gegenüber dem Kontaktelement 36 isoliert angeordnet ist. Koaxial um den Stift 64 ist das Federelement 39 in Form einer Schraubenfeder 65 angeordnet. Die Schraubenfeder 65 stützt sich einerseits an einem Kopf 66 des Stifts 64 und andererseits an einem in der Durchgangsöffnung 63 vorhandenen Radialvorsprung 67 ab, der beim Ausführungsbeispiel als Ringstufe ausgeführt ist. Die Schraubenfeder 65 ist auf Druck beansprucht und drückt den zweiten Kontaktträger 45 zum ersten Kontaktelement 35 hin. Wie bei allen anderen Ausführungsformen auch, sind die Federelemente 39 in Querrichtung Q jeweils auf beiden Seiten des Aufnahmebereichs 26 oder der beiden Aufnahmebereiche 26 einer Steckerbuchse 18 angeordnet.

Der zweite Kontaktträger 45 trägt das zweite Kontaktelement 36, dessen Kontaktflächenbereich 41 hier in Einsteckrichtung R kürzer ist als der Kontaktflächenbereich 41 des zugeordneten ersten Kontaktelements 35. Das zweite Kontaktelement 36 weist einen in Einsteckrichtung R und in Querrichtung Q gerade verlaufenden Kontaktflächenabschnitt 41 auf, wohingegen die Kontaktflächenabschnitte 41 der ersten Kontaktelemente 35 in Querrichtung Q konkav gewölbt sind.

Das Funktionsprinzip der zuvor erläuterten Ausführungsformen der Kontaktanordnungen 28 bzw. der Buchsenanordnungen 15 wird nachfolgend anhand der Figuren 8 und 9 erläutert.

In Figur 8 befinden sich die beiden Kontaktelemente 35, 36 einer Kontaktanordnung 28 in ihrer Ausgangslage, in der ihr Abstand in Bewegungsrichtung B angrenzend an den zweiten Abschnitt 26b des Aufnahmebereichs 26 kleiner ist, als der Durchmesser bzw. die Dicke des kleinsten Steckerkontaktstiftes 19. Beim Einstecken des Steckerkontaktstiftes 19 in die Steckerbuchse 18 werden die beiden Kontaktelemente gegen die Vorspannkraft der Federeinrichtung 38 in Bewegungsrichtung B auseinander gedrückt und liegen unter der Vorspannkraft von zwei entgegengesetzten Seiten am Steckerkontaktstift 19 an (Figur 9). Dadurch werden sowohl eine gut elektrische, wie auch eine gute mechanische Verbindung zwischen der Steckerbuchse 18 und dem Steckerkontaktstift 19 erreicht.

Die Vorspannkraft der Federeinrichtung 38 bei eingestecktem Steckerkontaktstift 19 ist so definiert, dass die Kraft zum Verschieben eines Steckerkontaktstiftes 19 oder eines Steckers 20 mit mehreren Steckerkontaktstiften 19 zumindest 15 N und maximal 60 N beträgt. Die Steck- und Ziehkraft schwankt dabei abhängig von dem Durchmesser bzw. der Dicke der Steckerkontaktstifte 19 eines Steckers 20.

Bei allen Ausführungsformen der Buchsenanordnung 15 mit den verschiedenen Kontaktanordnungen 28 kann eine Steckererkennungseinrichtung 70 vorhanden sein, wie sie lediglich nach Art eines Blockschaltbildes in den Figuren 8 und 9 dargestellt ist. Die Steckererkennungseinrichtung 70 ist vorzugsweise elektrisch mit dem ersten Kontaktelement 35 und dem zweiten Kontaktelement 36 einer oder mehrerer Steckerbuchsen 18 jeweils separat verbunden. Beim Ausführungsbeispiel besteht eine solche elektrische Verbindung mit den beiden Kontaktelementen 35, 36 der Einzelbuchse 51 sowie zumindest einer der beiden Doppelbuchsen 50.

Die beiden Kontaktelemente 35, 36 einer gemeinsamen Steckerbuchse 18 liegen bei nicht eingestecktem Stecker bzw. Steckerkontaktstift 19 elektrisch auf unterschiedlichen Potenzialen und sind elektrisch voneinander isoliert. Beim Einstecken des Steckerkontaktstiftes 19 wird ein Kurzschluss zwischen den beiden Kontaktelementen 35, 36 erzeugt. Diese Potenzial- bzw. Zustandsänderung kann durch die Steckererkennungseinrichtung 70 festgestellt werden. Beispielsweise kann eine Prüfspannung an den beiden Kontaktelementen 35, 36 anliegen. Ohne eingesteckten Steckerkontaktstift 19 fließt kein Strom. Sobald der Steckerkontaktstift 19 beide Kontaktelemente 35, 36 berührt, wird der Stromkreis geschlossen und es fließt ein Prüfstrom.

Über die Steckererkennungseinrichtung 70 kann somit auch erkannt werden, in welchen Steckerbuchsen 18 sich ein Steckerkontaktstift 19 befindet. Werden drei Steckerkontaktstifte 19 erkannt, kann die Steckererkennungseinrichtung 70 an das elektromedizinische Gerät ein Signal ausgeben, dass es sich bei dem angeschlossenen Instrument um ein monopolares Instrument mit einem Elektrodenanschluss und zwei Steueranschlüssen handelt. Werden hingegen nur zwei Steckerkontaktstifte 19 erkannt, kann auf ein bipolares Instrument geschlossen werden.

Somit kann die Steckererkennungseinrichtung 70 an das Gerät ein Steuersignal ausgeben, das den angeschlossenen Instrumententyp (monopolar oder bipolar) angibt. Außerdem kann dieses Steuersignal oder ein separates Signal als Freigabesignal verwendet werden, das dem Gerät anzeigt, dass ein Instrument angeschlossen ist und entsprechend an den Elektrodenanschlüssen die zum Betrieb des Instruments notwendige Hochfrequenzspannung angelegt werden kann.

Es versteht sich, dass die Formen und Konturen der verschiedenen Varianten der Kontaktanordnungen 28 abgewandelt und miteinander kombiniert werden können. Beispielsweise können die Kontaktflächenbereiche 41 der Kontaktelemente 35, 36 in Querrichtung Q konkav und/oder in Einsteckrichtung R konvex gewölbt ausgeführt sein. Die Kontaktflächenbereiche 41 können auch in Querrichtung Q und/oder in Einsteckrichtung R gerade verlaufen. Hierbei sind beliebige Kombinationen möglich.

Bei allen Ausführungsbeispielen kann nur das zweite Kontaktelement 36 bzw. nur der zweite Kontaktträger 45 oder alternativ beide Kontaktelemente 35, 36 bzw. beide Kontaktträger 44, 45 in Bewegungsrichtung B vorzugsweise linear verschiebbar geführt sein. In Abwandlung zu den beschriebenen Ausführungsformen könnten auch Schwenkbewegungen mit einer in Bewegungsrichtung B gerichteten Komponente vorgesehen werden.

Die Erfindung betrifft eine Buchsenanordnung 15 mit wenigstens einer Steckerbuchse 18. Jede Steckerbuchse 18 weist eine Kontaktanordnung 28 mit zwei elektrisch leitfähigen Kontaktelementen 35, 36 und einer Federeinrichtung 38 auf. Die beiden Kontaktelemente 35, 36 sind in einer Bewegungsrichtung B rechtwinklig oder schräg zu einer Einsteckrichtung R relativ zueinander bewegbar gelagert. Die Federeinrichtung 38 beaufschlagt ein oder beide Kontaktelemente 35, 36 in Bewegungsrichtung B. Über die Vorspannkraft der Federeinrichtung 38 sind die beiden Kontaktelemente 35, 36 aufeinander zu gedrückt oder gezogen. Bei in die Steckerbuchse 18 eingestecktem Steckerkontaktstift 19 werden die beiden Kontaktelemente in Bewegungsrichtung B aufeinander bewegt und liegen von unterschiedlichen Seiten am Steckerkontaktstift 19 an. Dadurch werden eine mechanische Klemmung und eine elektrische Kontaktierung hergestellt.

### Bezugszeichenliste:

- 15: Buchsenanordnung
- 16: Gehäuseteil
- 17: Buchsengehäuse
- 18: Steckerbuchse
- 19: Steckerkontaktstift
- 20: Stecker

- 23: Datenschnittstelle
- 24: Steckkontakt
- 25: Anschlussseite
- 26: Aufnahmebereich
- 26a: erster Abschnitt des Aufnahmebereichs
- 26b: zweiter Abschnitt des Aufnahmebereichs
- 26c: dritter Abschnitt des Aufnahmebereichs
- 27: Gehäuseöffnung
- 28: Kontaktanordnung
- 29: freies Ende des Steckerkontaktstifts
- 30: Aussparung
- 31: Buchsenteil

- 35: erstes Kontaktelement
- 36: zweites Kontaktelement
- 37: Anschlussfahne
- 38: Federeinrichtung
- 39: Federelement
- 40: Elastomerring
- 41: Kontaktflächenbereich

- 44: erster Kontaktträger
- 45: zweiter Kontaktträger
- 46: Führungseinrichtung
- 47: Schlitz
- 50: Doppelbuchse
- 51: Einzelbuchse

- 55: Schenkel
- 56: Querstück
- 57: Führungsaussparung
- 58: Führungsvorsprung
- 59: Querfortsatz
- 60: Isolator
- 61: Außenflächenbereich

- 63: Durchgangsöffnung
- 64: Stift
- 65: Schraubenfeder
- 66: Kopf
- 67: Radialvorsprung
- 68: Anschlagfläche
- 70: Steckererkennungseinrichtung

- A: Abstand
- B: Bewegungsrichtung
- Q: Querrichtung
- R: Einsteckrichtung

## Patentansprüche

1. Buchsenanordnung (15) für ein elektromedizinisches Gerät zum Anschluss eines elektromedizinischen, vorzugsweise chirurgischen Instruments,
mit wenigstens einer in einem Gehäuseteil (16) angeordneten Steckerbuchse (18), die ein elektrisches erstes Kontaktelement (35) und ein relativ zum ersten Kontaktelement (35) bewegliches elektrisches zweites Kontaktelement (36) aufweist, wobei die Kontaktelemente (35, 36) auf gegenüberliegenden Seiten eines Aufnahmebereichs (26) für einen Steckerkontaktstift (19) angeordnet sind,
mit einer Federeinrichtung (38), die das zweite Kontaktelement (36) der Steckerbuchse (18) mit einer zum ersten Kontaktelement (35) hin gerichteten Vorspannkraft beaufschlagt,
wobei der erste Kontaktträger (44) des ersten Kontaktelements (35) und/oder der zweite Kontaktträger (45) des zweiten Kontaktelements (36) in der Richtung B, quer zu einer Einsteckrichtung (R) des Steckerkontaktstifts (19) beweglich am Gehäuseteil (16) gelagert ist,
die beiden Kontaktträger (44, 45) eine Führungseinrichtung (46) aufweisen und unmittelbar aufeinander zu verschiebbar gelagert sind,
**dadurch gekennzeichnet,**
**dass** das erste Kontaktelement (35) auf einem elektrisch nicht leitfähigen ersten Kontaktträger (44) und/oder das zweite Kontaktelement (36) auf einem nicht leitfähigen elektrisch zweiten Kontaktträger (45) angeordnet ist,
**dass** die Führungseinrichtung (46) im Gehäuseteil (16) einen in Bewegungsrichtung B verlaufenden Schlitz (47) oder eine nutähnliche Aussparung aufweist, in die der zugeordnete Kontaktträger (44, 45) eingreift, und
**dass** der Kontaktträger (44, 45) zur Verdrehsicherung dieser zwei Abflachungen aufweist, mit denen er an den Schlitzwänden des Schlitzes (47) gleitend anliegt.

2. Buchsenanordnung nach Anspruch 1, **dadurch gekennzeichnet, dass** beide Kontaktelemente (35, 26) der Steckerbuchse (18) relativ zum Gehäuseteil (16) beweglich gelagert sind.

3. Buchsenanordnung nach Anspruch 1 oder 2, **dadurch gekennzeichnet, dass** die beiden Kontaktelemente (35, 36) der Steckerbuchse (18) elektrisch voneinander isoliert sind.

4. Buchsenanordnung nach einem der vorhergehenden Ansprüche, **dadurch gekennzeichnet, dass** eine Steckererkennungseinrichtung (70) vorhanden ist, die mit den beiden Kontaktelementen (35, 36) der Steckerbuchse (18) elektrisch verbunden ist.

5. Buchsenanordnung nach einem der vorhergehender. Ansprüche, **dadurch gekennzeichnet, dass** das erste Kontaktelement (35) auf einem elektrisch nicht leitfähigen ersten Kontaktträger (44) und/oder das zweite Kontaktelement (36) auf einem nicht leitfähigen elektrisch zweiten Kontaktträger (45) angeordnet ist.

6. Buchsenanordnung nach Anspruch 1, **dadurch gekennzeichnet, dass** der erste Kontaktträger (44) des ersten Kontaktelements (35) und/oder der zweite Kontaktträger (45) des zweiten Kontaktelements (36) gemeinsam mit dem Gehäuseteil (16) die Führungseinrichtung (46) zur verschiebbaren Lagerung des betreffenden Kontaktträgers (44, 45) bilden.

7. Buchsenanordnung nach einem der Ansprüche 1 oder 6, **dadurch gekennzeichnet, dass** die Federeinrichtung (38) zumindest einen der Kontaktträger (44, 45) beaufschlagt.

8. Buchsenanordnung nach einem der Ansprüche 1 oder 6 bis 7, **dadurch gekennzeichnet, dass** sich die Federeinrichtung (38) am ersten Kontaktträger (44) des ersten Kontaktelements (36) und/oder am zweiten Kontaktträger (45) des zweiten Kontaktelements (36) abstützt.

9. Buchsenanordnung nach einem der Ansprüche 1 oder 6 bis 8, **dadurch gekennzeichnet, dass** sich die Federeinrichtung (38) unmittelbar am ersten Kontaktelement (35) oder am Gehäuseteil (16) und am zweiten Kontaktträger (45) des zweiten Kontaktelements (36) abstützt.

10. Buchsenanordnung nach einem der Ansprüche 1 oder 6 bis 9, **dadurch gekennzeichnet, dass** zumindest zwei Steckerbuchsen (18) mit jeweils einem ersten und einem zweiten Kontaktelement (35, 36) vorhanden sind, wobei die beiden ersten Kontaktelemente (35) und/oder die beiden zweiten Kontaktelemente (36) auf einem gemeinsamen Kontaktträger (44, 45) angeordnet sind.

11. Buchsenanordnung nach einem der vorhergehenden Ansprüche, **dadurch gekennzeichnet, dass** wenigstens eine Steckerbuchse (18) als Doppelbuchse (50) mit zwei in einer Einsteckrichtung (R) nebeneinander angeordneten Aufnahmebereichen (26) für den Steckerkontaktstift (19) aufweist.

12. Buchsenanordnung nach einem der vorhergehenden Ansprüche, **dadurch gekennzeichnet, dass** das erste Kontaktelemente (35) und/oder das zweite Kontaktelement (36) quer zu einer Einsteckrichtung (R) konkav gewölbt ist.

13. Buchsenanordnung nach einem der vorhergehenden Ansprüche, **dadurch gekennzeichnet, dass** das erste Kontaktelement (35) und/oder das zweite Kontaktelement (36) um eine quer zu einer Einsteckrichtung (R) verlaufenden Achse drehbar gelagert sind.

## Claims

1. Socket arrangement (15) for an electromedical device for the connection of an electromedical, preferably surgical instrument, comprising
at least one plug connector socket (18) arranged in a housing part (16), the plug connector socket having an electrical first contact element (35) and an electrical second contact element (36) configured to move relative to the first contact element (35), wherein the contact elements (35, 36) are arranged on opposite sides of a receiving region (26) for a plug contact pin (19),
a spring arrangement (38), configured to apply a pretensioning force to the second contact element (36) of the plug connector socket (18), the pretensioning force being directed toward the first contact element (35),
wherein the first contact holder (44) of the first contact element (35) and/or the second contact holder (45) of the second contact element (36) are supported on the housing part (16) so as to be movable in the direction B transverse to a plug-in direction (R) of the plug contact pin (19),
the two contact holders (44, 45) comprise a guide arrangement (46) and are supported so as to be movable back and forth directly adjacent to each other,
**characterized,**
**in that** the first contact element (35) is arranged on an electrically non-conductive first contact holder (44) and/or the second contact element (36) is arranged on an electrically non-conductive second contact holder (45),
**in that** the control device (46) in the housing part (16) has a slit (47), extending in the movement direction B, or a groove-like recess, in which the associate in that to prevent a twisting of the respective contact holder (44, 45) this has two flat areas with which it abuts in a sliding manner against the slit walls of the slit (47).

2. Socket arrangement according to claim 1, **characterized in that** both contact elements (35, 36) of the plug connector socket (18) are supported so as to be movable relative to the housing part (16).

3. Socket arrangement according to claim 1 or 2, **characterized in that** both contact elements (35, 36) of the plug connector socket (18) are electrically insulated from each other.

4. Socket arrangement according to any one of the preceding claims, **characterized in that** a plug detection arrangement (70), which is electrically connected with the two contact elements (35, 36) of the plug connector socket (18), is provided.

5. Socket arrangement according to any one of the preceding claims, **characterized in that** the first contact element (35) is arranged on an electrically non-conductive first contact holder (44) and/or the second contact element (36) is arranged on an electrically non-conductive second contact holder (45).

6. Socket arrangement according to claim 1, **characterized in that** the first contact holder (44) of the first contact element (35) and/or the second contact holder (45) of the second contact element (36) form the guide arrangement (46) together with the housing part (16) for the movable support of the respective contact holder (44, 45).

7. Socket arrangement according to claims 1 or 6, **characterized in that** the spring arrangement (38) is configured to act on at least one of the contact holders (44, 45).

8. Socket arrangement according to any one of claims 1 or 6 - 7, **characterized in that** the spring arrangement (38) is supported by the first contact holder (44) of the first contact element (36) and/or by the second contact holder (45) of the second contact element (36).

9. Socket arrangement according to any one of claims 1 or 6 to 8, **characterized in that** the spring arrangement (38) is directly supported by the fist contact element (35) or by the housing part (16) and by the second contact holder (45) of the second contact element (36).

10. Socket arrangement according to any one of claims 1 or 6 to 9, **characterized in that** at least two plug connector sockets (18) having a first and a second contact element (35, 36) are provided, wherein both first contact elements (35) and/or both second contact elements (36) are arranged on a shared contact holder (44, 45).

11. Socket arrangement according to any one of the preceding claims, **characterized in that** at least one plug connector socket (18) is configured as a double socket (50) with two receiving regions (26) for the plug contact pin (19), the receiving regions being arranged next to each other in a plug-in direction (R).

12. Socket arrangement according to any one of the preceding claims, **characterized in that** the first contact element (35) and/or the second contact element (36) are concavely curved in a direction transverse to a plug-in direction (R).

13. Socket arrangement according to any one of the preceding claims, **characterized in that** the first contact element (35) and/or the second contact element (36) are supported so as to be rotatable about an axis extending in a direction transverse to a plug-in direction (R).

## Revendications

1. Agencement de douille (15) pour un appareil électromédical, destiné au raccordement d'un instrument électromédical, de préférence chirurgical,
comprenant au moins une prise femelle (18), disposée dans un élément de boîtier (16), qui présente un premier élément de contact (35) électrique et un deuxième élément de contact (36) électrique, déplaçable par rapport au premier élément de contact (35), les éléments de contact (35, 36) étant disposés sur des côtés opposés d'une zone de réception (26) pour une broche de contact de prise (19),
comprenant un dispositif à ressort (38) qui sollicite le deuxième élément de contact (36) de la prise femelle (18) avec une force de précontrainte dirigée vers le premier élément de contact (35),
dans lequel le premier porte-contact (44) du premier élément de contact (35) et/ou le deuxième porte-contact (45) du deuxième élément de contact (36) est/sont monté(s) sur l'élément de boîtier (16) avec possibilité de déplacement dans la direction B, perpendiculairement à une direction d'enfichage (R) de la broche de contact de prise (19),
les deux porte-contacts (44, 45) présentent un dispositif de guidage (46) et sont montés de façon à pouvoir être coulissés directement l'un vers l'autre,
**caractérisé en ce que**
le premier élément de contact (35) est disposé sur un premier porte-contact (44) non conducteur électrique et/ou le deuxième élément de contact (36) est disposé sur un deuxième porte-contact (45) non conducteur électrique,
que le dispositif de guidage (46) dans l'élément de boîtier (16) présente une fente (47), s'étendant dans la direction de déplacement B, ou un évidement en forme de rainure dans lequel s'engage le porte-contact (44, 45) associé, et
que le porte-contact (44, 45) présente, en vue du blocage en rotation de celui-ci, deux méplats avec lesquels il est appliqué de façon glissante contre les parois de fente de la fente (47).

2. Agencement de douille selon la revendication 1, **caractérisé en ce que** les deux éléments de contact (35, 36) de la prise femelle (18) sont montés avec possibilité de déplacement par rapport à l'élément de boîtier (16).

3. Agencement de douille selon la revendication 1 ou 2, **caractérisé en ce que** les deux éléments de contact (35, 36) de la prise femelle (18) sont isolés électriquement l'un de l'autre.

4. Agencement de douille selon l'une des revendications précédentes, **caractérisé en ce qu'**il est prévu un dispositif de détection de prise (70) qui est connecté électriquement aux deux éléments de contact (35, 36) de la prise femelle (18).

5. Agencement de douille selon l'une des revendications précédentes, **caractérisé en ce que** le premier élément de contact (35) est disposé sur un premier porte-contact (44) non conducteur électrique et/ou le deuxième élément de contact (36) est disposé sur un deuxième porte-contact (45) non conducteur électrique,

6. Agencement de douille selon la revendication 1, **caractérisé en ce que** le premier porte-contact (44) du premier élément de contact (35) et/ou le deuxième porte-contact (45) du deuxième élément de contact (36) forment conjointement avec l'élément de boîtier (16) le dispositif de guidage (46) destiné au montage coulissant du porte-contact (44, 45) respectif.

7. Agencement de douille selon l'une des revendications 1 ou 6, **caractérisé en ce que** le dispositif à ressort (38) sollicite au moins un des porte-contacts (44, 45).

8. Agencement de douille selon l'une des revendications 1 ou 6 à 7, **caractérisé en ce que** le dispositif à ressort (38) est en appui sur le premier porte-contact (44) du premier élément de contact (36) et/ou sur le deuxième porte-contact (45) du deuxième élément de contact (36).

9. Agencement de douille selon l'une des revendications 1 ou 6 à 8, **caractérisé en ce que** le dispositif à ressort (38) est en appui directement sur le premier élément de contact (35) ou sur l'élément de boîtier (16) et sur le deuxième porte-contact (45) du deuxième élément de contact (36).

10. Agencement de douille selon l'une des revendications 1 ou 6 à 9, **caractérisé en ce qu'**il est prévu au moins deux prises femelles (18) comprenant chacune un premier et un deuxième élément de contact (35, 36), sachant que les deux premiers éléments de contact (35) et/ou les deux deuxièmes éléments de contact (36) sont disposés sur un porte-contact (44,45) commun.

11. Agencement de douille selon l'une des revendications précédentes, **caractérisé en ce qu'**au moins une prise femelle (18), en tant que prise double (50), présente deux zones de réception (26) pour la broche de contact de prise (19) qui sont disposées l'une à côté de l'autre dans une direction d'enfichage (R).

12. Agencement de douille selon l'une des revendications précédentes, **caractérisé en ce que** le premier élément de contact (35) et/ou le deuxième élément de contact (36) est/sont incurvé(s) sous une forme concave, perpendiculairement à une direction d'enfichage (R).

13. Agencement de douille selon l'une des revendications précédentes, **caractérisé en ce que** le premier élément de contact (35) et/ou le deuxième élément de contact (36) sont montés avec possibilité de rotation autour d'un axe qui s'étend perpendiculairement à une direction d'enfichage (R).
